# EUROPEAN PATENT SPECIFICATION

(11) **EP 4 251 018 B1**
(45) Date of publication and mention of the grant of the patent: **30.10.2024**
(21) Application number: 21823672.7
(22) Date of filing: 26.11.2021
(51) Int. Cl.: A47J 43/14, A23J 1/09

(54) **APPARATUS FOR SHELLING EGGS**
VORRICHTUNG ZUM SCHÄLEN VON EIERN
APPAREIL POUR ÉCALER DES OEUFS

(30) Priority: 27.11.2020 IT 202000028796
(43) Date of publication of application: 04.10.2023
(73) Proprietor: Moba Group B.V., 3771 VE Barneveld (NL)
(72) Inventor: PESCHI, Leopoldo, 31100 Treviso (IT); GUALTIERI, Fabio, 31100 Treviso (IT); BUISMAN, Paul, 3771 VE Barneveld (NL)
(74) Representative: V.O.
(86) International application number: PCT/IB2021/060997
(87) International publication number: WO 2022/113006

(56) References cited:
- WO-A2-2011/119825
- CN-A- 108 782 940
- US-A- 3 420 743

## Description

### TECHNICAL FIELD

This invention relates to an apparatus for shelling eggs and especially for obtaining a mixed product, yolk and albumen, or which makes yolk and albumen available separately, where the shelling product allows a longer shelf life to be obtained, other treatment conditions being equal, compared to prior art solutions.

### BACKGROUND ART

In the field of treatment of food products, both the need to obtain a long shelf life and to obtain products with organoleptic properties close to those of the fresh product are strongly felt.

In the field of treatment of eggs, long shelf life is achieved by adjusting the pasteurization parameters.

In other words, the product to be pasteurized, be it a mixture of yolk and albumen or pure albumen, is made to touch a hot surface from which it receives the pasteurization heat.

The pasteurization temperature must be maintained, depending on the product to be pasteurized, in a range substantially between 57°C and 72°C to avoid degrading the product at higher temperatures or to avoid obtaining insufficient pasteurization by using lower temperatures.

The aim of pasteurization is to reduce the bacterial load present in the product to be pasteurized, therefore, together with the temperature, a key parameter of the pasteurization process is the duration of the isothermal period, that is, the duration of the product being kept at the pasteurization temperature.

Within the above-mentioned temperature limits, a longer isothermal period determines a greater reduction of the bacterial load, therefore, depending on the intended use of the final product, higher pasteurization temperatures are chosen combined with shorter isothermal periods or, vice versa, longer isothermal periods combined with lower temperatures.

According to the bacterial load contained in the product to be pasteurized, the pasteurization temperature and the relative isothermal period will be determined. Higher temperatures with shorter isothermal periods benefit productivity, however lower pasteurization temperatures and longer isothermal periods favour the organoleptic qualities of the resulting product.

In general, the organoleptic qualities of the resulting product are better the less rigid the thermal pasteurization treatment is, that is, the lower the temperature and the shorter the isothermal period.

WO2011/119825 addresses a system for providing automated inspection, removal and tracking of eggs advancing along an egg conveying apparatus and prior to an egg breaking stage.

US3,420,743 provides an improved machine for harvesting vaccines or similar biologicals from parts of embryonated eggs which have been inoculates with various biologicals, such as a virus and the like.

### SUMMARY OF THE INVENTION

The problem underlying the invention is therefore that of improving the shelf life of a product deriving from shelling of eggs, be it a mixture of egg albumen and yolk or only albumen or only yolk, obtaining organoleptic qualities of the product which are significantly comparable with those of the fresh product.

The task of an egg shelling apparatus according to the invention is therefore to solve this problem.

Within this aim, an object of the invention is to provide an apparatus for shelling eggs which can guarantee a bacterial load of the final product, with the same raw material, which is considerably lower than that obtainable by means of traditional plants and processes.

Within this task, an aim of the invention is to provide an apparatus for shelling eggs which, for the same quality of the final product, allows shorter production times to be obtained with respect to prior art plants and processes.

Another aim of the invention is to provide an apparatus for shelling eggs which is simple to implement and easy to use.

This aim, as well as these and other aims which will emerge more fully below, are attained by an apparatus according to the appended independent claim.

Detailed features of an apparatus for shelling eggs according to the invention are indicated in the dependent claims.

Further features and advantages of the invention will emerge more fully from the description of a preferred but not exclusive embodiment of an apparatus for shelling eggs, illustrated by way of non-limiting example in the accompanying drawings, in which:

### BRIEF DESCRIPTION OF THE DRAWINGS

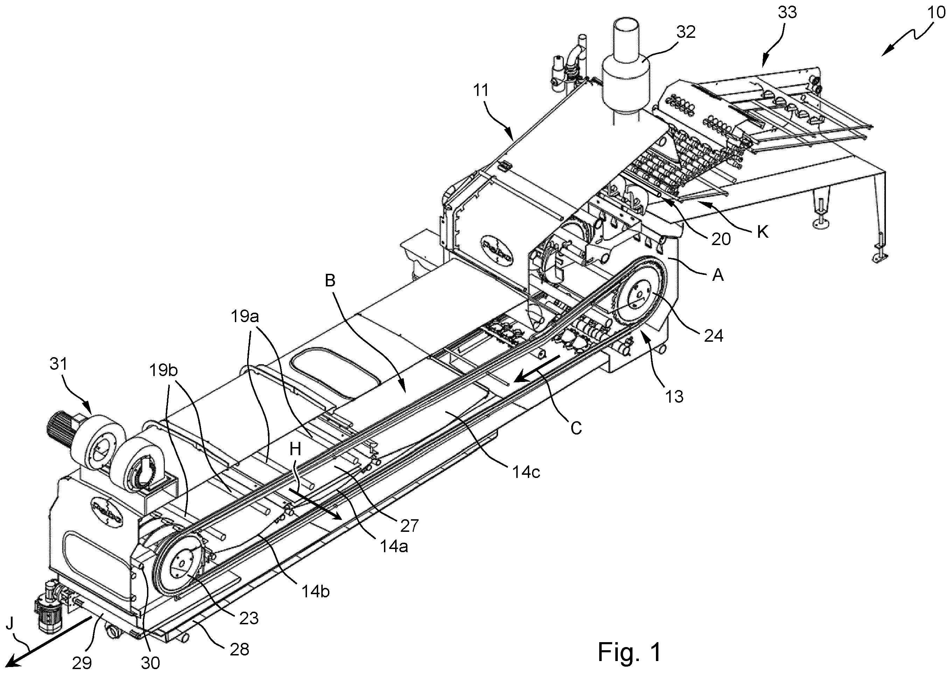

- Figure 1 shows a diagram of an apparatus for treating eggs according to the invention;
- Figure 2 shows a detail of an apparatus for treating eggs according to the invention, relating to a piston filter, in a median section;
- Figure 3 illustrates a detail of an apparatus for treating eggs according to the invention, relating to a module for transporting yolk and albumen;

- Figure 4 shows a detail of an apparatus for treating eggs according to the invention, relating to a series of modules of Figure 3;
- Figure 5 is a perspective view of a detail of an apparatus for treating eggs according to the invention, relating to a shelling device and a transport device, with some parts removed so as to better highlight others.

### DETAILED DESCRIPTION

With particular reference to the accompanying drawings, the reference number 10 denotes in its entirety an apparatus for shelling eggs which comprises:
- a casing 11 defining a shelling compartment A and an unloading compartment B;
- a shelling device 12 housed in the shelling compartment A and configured to open egg shells;
- an unloading device 13 housed in the unloading compartment B and comprising at least one hopper member 14a, 14b, 14c suitable for receiving and unloading a shelling product consisting of albumen, yolk or a mixture of albumen and yolk;
- a transport device 15 comprising conveyors 16, 17 configured to separately transport albumen and yolk from the shelling device 12 to the unloading device 13;
- sanitizing devices 18, 19a, 19b, 20, 30 housed in the casing 11 and adapted to emit ultraviolet radiation and which comprise a first sanitizing device 18, and possibly an accessory sanitizing device 19b, positioned to irradiate operating surfaces of the hopper member 14a, 14b, respectively, to reduce a bacterial load on the operating surfaces of the latter, which consist of surfaces that come into contact with the yolk and/or albumen during the operation of the apparatus.

The sanitizing devices 18, 19a, 19b, 20, 30 can comprise one or more ultraviolet lamps which can be tubular.

For example, these ultraviolet radiations can have a power of between 100 and 2500 µW/cm² for a time between 10 and 60 seconds, to counteract bacterial proliferation and, moreover, each shelling device is sanitized, following each shelling, for example by spraying with a spray of ozonated water or with an aqueous solution of hydrogen peroxide, for example at 1.4%.

Thanks to the irradiation carried out by the sanitizing devices 18, 19a, 19b, 20, 30, in an apparatus 10 according to the invention it is possible to significantly reduce the bacterial load contained in the shelling product so that an egg treatment plant comprising an apparatus 10 allows, with the same other functional parameters, a product to be obtained with a lower final bacterial load and, therefore, with a longer shelf life.

Moreover, in an egg treatment plant comprising an apparatus 10 according to the invention, it is possible to adopt lower pasteurization temperatures and/or lower times at the pasteurization temperature, because the bacterial load to be reduced is lower.

Thus, the use of an apparatus according to the invention in a plant for treating eggs allows, with the same other functional parameters, to obtain a product with better organoleptic qualities than prior art plants.

The sanitizing devices 18, 19a, 19b, 20, 30 can comprise a third sanitizing device 20 positioned to irradiate working parts of the shelling device 12 where these working parts include parts able to come into contact with an egg or with the shell and/or with the shelled egg during operation of the apparatus 10, this eliminating or reducing any contamination of the shell and therefore of the shelling product, reducing its bacterial load.

The shelling device 12 comprises at least one opening member 34, of a traditional type and configured to open an egg shell and cause the yolk and albumen to fall. The conveyors 16, 17 comprise:
- at least a first conveyor 16 having a plurality of seats each adapted to house yolk;
- at least a second conveyor 17 having a plurality of seats each suitable for housing albumen.

Each opening member 34 is placed above the first conveyor 16 to discharge a yolk for each seat of the latter.

The first conveyor 16 is placed above the second conveyor 17 to discharge albumen into the seats of the second conveyor 17.

The said operating surfaces of the conveyors 16, 17 comprise said seats which, therefore, are irradiated by ultraviolet radiation during the operation of the apparatus 10, to reduce any bacterial load present on them.

The first sanitizing device 18 are positioned in such a way as to irradiate said seats before they are engaged respectively by a yolk and by an albumen, during the operation of the apparatus 10.

In this way the possibility of contamination, or its extent, of the shelling product received by the shelling device 12 is limited to the full.

The first sanitizing device 18 can be placed side by side with the shelling device 12 and, in particular, it can be placed upstream of the latter with respect to a direction of advancement C of the seats of the conveyors 16 and 17, to sanitize the latter just before being filled.

The conveyors 16, 17 may comprise:
- a plurality of modules 22, each comprising at least one of the seats, and which can be connected in sequence to form respectively a first chain member and a second chain member, which are closed in a ring;
- a first transmission unit 23 and a second transmission unit 24 to which the chain members are connected and between which the latter extend, defining a forward section D and a return section E of the chain members which extend from the shelling compartment A to the unloading compartment B.

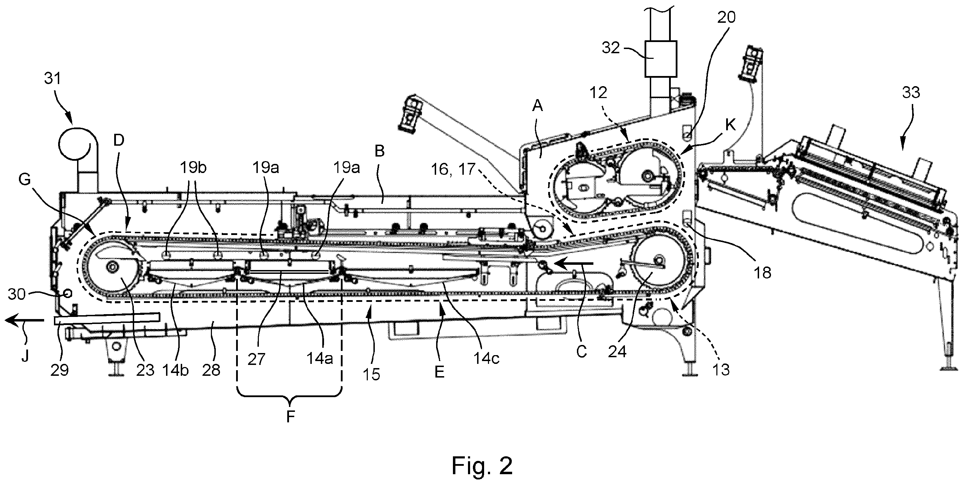

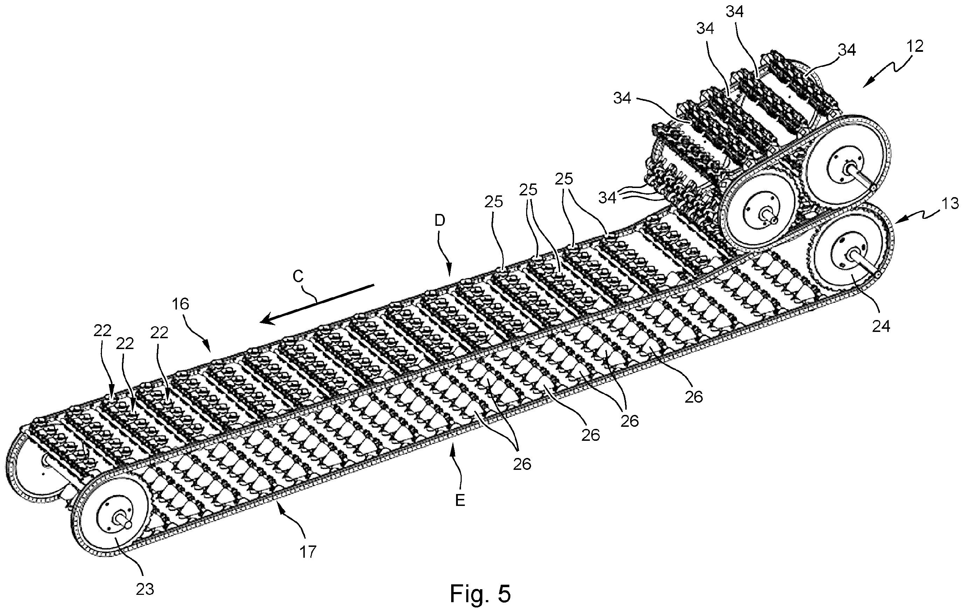

For example, Figure 5 illustrates a shelling device 12 and an unloading device 13, which in Figure 2 are schematized by a dashed line.

The unloading device 13 of Figure 5 has some parts removed to facilitate their intelligibility.

In Figure 5, in particular, the conveyors 16 and 17 are seen, where the first conveyor 16 is seen in the forward section D where it hides the second conveyor 17 which is under it.

On the contrary, in the return section E the second conveyor 17 is visible and hides the first conveyor 16 which is under it.

In the example of Figure 5 it can be seen how the conveyors 16 and 17 are integrated in a single product which has a plurality of modules 22, which form lines and rows respectively transversal and longitudinal with respect to the direction of advancement C.

The first chain member and the second chain member can constitute a single product, that is, consist of a single chain member.

The ultraviolet lamps of the sanitizing devices 18, 19a, 19b, 20, 30 can extend transversely to the direction of advancement C to irradiate a plurality of modules 22 side by side which, during operation of the system, move along the direction of advancement C.

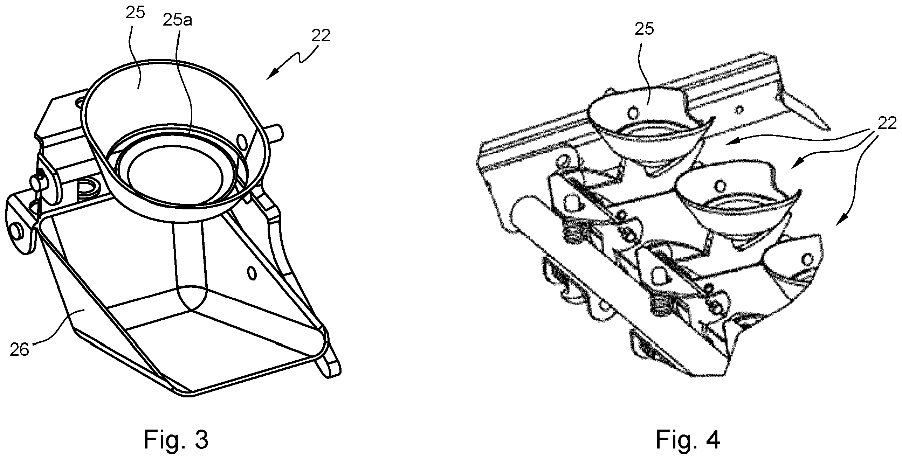

The seats of the conveyors 16, 17 can be defined respectively by a first spoon element 25 and a second spoon element 26, for example like those illustrated in Figures 3, 4 and 5.

Each module 22 can comprise a first spoon element 25 and a second spoon element 26 which can be reciprocally positioned in such a way that, along the forward section D, the first spoon element 25 is positioned above the second spoon element 26, when the apparatus 10 is in use.

The first spoon element 25 can be configured in such a way as to retain a yolk and discharge the albumen of an egg.

For example, it can have at least one opening or holes 25a positioned and configured not to allow the passage of a whole yolk but to allow the passage of the albumen that surrounds it.

The second spoon element 26 can be configured so as to receive an albumen unloaded from the first spoon element 25 of the same module 22.

The unloading device 13 can comprise:
- an emptying member operable to interfere with the second conveyor 17 in a first unloading position F of the unloading compartment B;
- a first hopper member 14a of the at least one hopper member 14a, 14b, 14c which is positioned so that, in use, it is under the second conveyor 17 in the first unloading position F to receive the contents of the seats of the second conveyor 17.

The emptying member can be configured to cause, following its actuation, a fall of the contents of the seats of the first conveyor 16 when these pass through the first unloading position F during the operation of the apparatus 10.

For example, the emptying member can be configured to rotate the second spoon 26 in such a way as to cause it to overturn to make its contents fall into the first hopper member.

The emptying member and the conveyors 16 and 17 can be of a traditional type and, therefore, not further described or illustrated.

The apparatus 10 may comprise a first filtering device 27 positioned in such a way that in use it is located above the first hopper member 14a to filter and retain eggshell fragments and allow the passage of the shelling product.

The second sanitizing device 19 can face the first filtering device 27 and/or the first hopper member 14a to reduce any bacterial load present on them.

The unloading device 13 can comprise a second hopper member 14b and/or a third hopper member 14c which can be housed in the unloading compartment B and positioned, with respect to the conveyors 16 and 17, so that when the apparatus is in use they are under conveyors 16 and 17.

The second sanitizing device 19b can face the second hopper member 14b to sanitize it.

The unloading device 13 can be configured to cause the falling of shelling product from the seats of both the conveyors 16 and 17 into the second hopper member 14b, to simultaneously collect both the yolk and the albumen together.

Moreover, the unloading device 13 can be configured to cause the falling of shelling product selectively from specific seats of the conveyors 16 and 17 into the third hopper member 14c, to collect waste yolks and/or albumen.

In this case, the selection of said specific seats can be carried out by an operator or by a selection device, possibly equipped with a viewing system facing said seats, which is integrated or connected with the unloading device 13 to operate it so as to unload their contents from said specific seats when such content is identified as to be discarded.

The unloading device 13 can comprise:
- a tank 28 positioned in the unloading compartment B in such a way that in use it is under the first conveyor 16 to receive the shelling product from the latter;
- a refrigeration device, not shown, thermally connected to the tank 28 to cool it to a temperature not higher than 15°C and preferably between 0°C and 4 C, for example by means of a cooling-jacket with recirculation of refrigerant associated with the tank 28.

The apparatus 10 can be configured to unload the shelling product from the first conveyor 16 into a second unloading position G of the unloading compartment B. The apparatus 10 can comprise a second filtering device 29 positioned in such a way as to receive shelling product from the first conveyor 16 unloaded in the second unloading position G, to filter and retain eggshell fragments and allow the passage of the shelling product.

The apparatus 10 can comprise a fourth sanitizing device 30 of said sanitizing devices 18, 19a, 19b, 20, 30 which faces the second filtering device 29 to reduce any bacterial load present on it.

The first filtering device 27 and/or the second filtering device 29, respectively, can comprise a mesh or perforated conveyor belt which can be operated to drag, in an ejection direction H, J, fragments of eggshell filtered during the operation of the apparatus 10.

The ejection direction H of the first filtering device 27 can be transversal and preferably perpendicular to the direction of advancement C.

The ejection direction H of the second filtering device 29 can be parallel to the direction of advancement C where the second unloading position G is located at one end of the transport device 15 distal to the shelling device 12.

The apparatus 10 may comprise ventilation means which are in aeraulic communication with the interior of the casing 11 to force a flow of gas into the latter.

These ventilation means may comprise or consist of one or more fans 31.
said gas is optionally filtered air or a gaseous mixture containing ozone.

Said filtered air can preferably consist of a flow of air filtered by means of a HEPA filter which can be fixed to the fans 31 to be crossed by the flow of air forced by the latter.

The apparatus 10 can comprise a suction unit 32 which is in aeraulic connection with the interior of the casing 11 and is configured to suck a gas contained in the latter.

In particular, the apparatus 10 is preferably configured to operate the ventilation means and/or the suction unit 32 in such a way that the inside of the casing 11 is in depression with respect to the relative environment outside if the gas introduced by the ventilation means in the casing 11 includes ozone.

The apparatus 10 may also comprise a loading device 33 configured to load eggs into the shelling device 12 through an inlet compartment K of the casing 11.

The inlet compartment K can be provided at a first end of the casing 11 where at a second end of the same, distal from the first end with respect to the direction of advancement C, an ejection opening L can be provided through which the second filtering device 29 can eject waste material from the casing 11.

The ultraviolet lamps of the first sanitizing device 19a and of the second sanitizing device 19b are preferably placed above the sides of the first hopper member 14a and of the second hopper member 14b respectively, for example in such a way that these lamps are spaced along the direction of advancement C so as to leave a free space between them for the falling of shelling product into the first and second hopper members 14a, 14b.

It is therefore understood how an apparatus according to the invention achieves the intended aims and objectives by improving the shelf life of a product deriving from the shelling of eggs, be it a mixture of albumen and yolk or only albumen or only yolk, obtaining organoleptic qualities of the product which are significantly comparable with those of the fresh product.

Furthermore, an apparatus for shelling eggs according to the invention allows a bacterial load of the final product to be guaranteed, with the same raw material, which is considerably lower than that obtainable by means of prior art systems and processes. Furthermore, an apparatus according to the invention allows, with the same quality of the final product, shorter production times to be obtained with respect to prior art plants and processes.

The invention thus conceived is susceptible to numerous modifications and variations, all of which fall within the scope of protection of the attached claims.

Where the operating and technical features mentioned are followed by signs or reference numbers, the signs or reference numbers have been used only with the aim of increasing the intelligibility of the description and claims themselves and, consequently, they do not constitute in any way a limitation to the interpretation of each element identified, purely by way of example, by the signs or reference numerals.

## Claims

1. Apparatus (10) for shelling eggs which includes:
- a casing (11) defining a shelling compartment (A) and an unloading compartment (B);
- a shelling device (12) housed in said shelling compartment (A) and configured to open egg shells;
- an unloading device (13) housed in said unloading compartment (B) and comprising at least one hopper member (14a, 14b, 14c) suitable for receiving and unloading a shelling product consisting of albumen, yolk or a mixture of albumen and yolk;
- a transport device (15) comprising conveyors (16, 17) configured to separately transport albumen and yolk from said shelling device (12) to said unloading device (13);
wherein said shelling device (12) comprises at least one opening member (34) configured to open an egg shell and cause the yolk and albumen to fall;
**characterized in that** the apparatus includes:
- sanitizing devices (18, 19a, 19b, 20, 30) housed in said casing (11) adapted to emit ultraviolet radiation and which comprise a first sanitizing device (18, 19a) positioned to irradiate operating surfaces of said at least one hopper organ (14a , 14b, 14c), to reduce a bacterial load on said operating surfaces, wherein said operating surfaces are surfaces that come into contact with yolk and/or albumen during the operation of said apparatus (10);
said conveyors (16, 17) comprise:
- at least a first conveyor (16) having a plurality of seats each adapted to house a yolk;
- at least a second conveyor (17) having a plurality of seats each suitable for housing albumen;
wherein each of said opening member (34) is arranged above one of said at least one first conveyor (16) to unload a yolk for each seat of said first conveyor (16);
wherein each of said at least one first conveyor (16) is arranged on top of one of said at least one second conveyor (17) to unload albumen into the seats of said second conveyor (17);
the operating surfaces of said conveyors (16, 17) comprise said seats; said first sanitizing device (18, 19a) also being positioned in such a way as to irradiate said seats before they are engaged respectively by a yolk and an albumen, during the operation of said apparatus (10).

2. Apparatus (10) according to claim 1 wherein said sanitizing devices (18, 19b, 20, 30) comprise a third sanitizing device (20) positioned to irradiate working parts of said shelling device (12) wherein said working parts comprise parts able to come into contact with an egg during the operation of said apparatus (10).

3. Apparatus (10) according to any of the previous claims wherein said first sanitizing device (18) is arranged side by side to said shelling device (12) and arranged upstream of the latter with respect to a direction of advancement (C) of said seats, to sanitize the latter just before being filled.

4. Apparatus according to one of the preceding claims wherein said unloading device (13) comprises
- an emptying member operable to interfere with said second conveyor (17) in a first unloading position (F) of said unloading compartment (B); said emptying member being configured to cause, following its actuation, a fall of the contents of the seats of said first conveyor (16) when they pass through said first unloading position (F) during the operation of said apparatus (10);
- a first hopper member (14a) of said at least one hopper member (14a, 14b, 14c) which is positioned so that, in use, it is under said second conveyor (17) in said first unloading position (F) to receive the contents of the seats of said second conveyor (17).

5. Apparatus (10) according to the preceding claim which comprises a first filtering device (27) positioned so that, in use, it is above said first hopper member (14a) to filter and retain eggshell fragments and allow the passage of said shelling product.

6. Apparatus (10) according to the previous claim wherein a second sanitizing device (19a) of said sanitizing devices (18, 19a, 19b, 20, 30) faces said first filtering device (27) and said first hopper member (14a) to reduce any bacterial load present on them.

7. Apparatus (10) according to one of the preceding claims wherein said unloading device (13) comprises:
- a tank (28) arranged in said unloading compartment (B) in such a way that in use it is under said first conveyor (16) to receive said shelling product from the latter;
- a refrigeration device thermally connected to said tank (28) to cool it to a temperature not higher than 15° C and preferably between 0° C and 4° C, for example by means of a cooling-jacket with recirculation of refrigerant associated with the tank (28).

8. Apparatus (10) according to the preceding claim configured for unloading said shelling product from said first conveyor (16) into a second unloading position (G) of said unloading compartment (B); said apparatus (10) comprising a second filtering device (29) arranged in such a way as to receive shelling product from said first conveyor (16) unloaded into said second unloading position (G), to filter and retain eggshell fragments and allowing the passage of said shelling product.

9. Apparatus (10) according to the preceding claim which comprises a fourth sanitizing device (30) of said sanitizing devices (18, 19a, 19b, 20, 30) which faces said second filtering device (29) to reduce any bacterial load present on it.

10. Apparatus (10) according to one of claims 5 or 6 and/or according to one of claims 8 or 9 wherein said first filtering device (27) and/or said second filtering device (29), respectively, comprises a mesh or perforated conveyor belt operable to drag, in an ejection direction (H, J), fragments of eggshell filtered during the operation of said apparatus (10).

11. Apparatus (10) according to one of the preceding claims which comprises ventilation means which are in aeraulic communication with the interior of said casing (11) to force a gas to flow into the latter.

12. Apparatus according to claim 11, wherein said gas is filtered air or a gaseous mixture containing ozone; said filtered air consisting of a flow of air filtered by a HEPA filter.

13. Apparatus (10) according to one of the preceding claims comprising a suction unit (32) which is in aeraulic connection with the interior of said casing (11) and is configured to suck a gas contained in the latter.

## Patentansprüche

1. Vorrichtung (10) zum Schälen von Eiern mit:
- einem Gehäuse (11), das eine Schälkammer (A) und eine Entladekammer (B) definiert;
- einer Schälvorrichtung (12), die in dem Schälkammer (A) untergebracht und dazu konfiguriert ist, Eierschalen zu öffnen;
- einer Entladevorrichtung (13), die in dem Entladekammer (B) untergebracht ist und mindestens ein Behälterelement (14a, 14b, 14c) umfasst, das zum Aufnehmen und Entladen von einem Schälprodukt geeignet ist, das aus Eiweiß, Eigelb oder einer Mischung aus Eiweiß und Eigelb besteht;
- einer Transportvorrichtung (15) mit Förderern (16, 17), die dazu konfiguriert sind, Eiweiß und Eigelb getrennt von der Schälvorrichtung (12) zu der Entladevorrichtung (13) zu transportieren;
wobei die Schälvorrichtung (12) mindestens ein Öffnungselement (34) umfasst, das dazu konfiguriert ist, eine Eierschale zu öffnen und das Herabfallen des Eigelbs und des Eiweißes zu bewirken;
**dadurch gekennzeichnet, dass** die Vorrichtung Folgendes aufweist:
- Desinfektionsvorrichtungen (18, 19a, 19b, 20, 30), die in dem Gehäuse (11) untergebracht sind, zum Emittieren ultravioletter Strahlung geeignet sind, und die eine erste Desinfektionsvorrichtung (18, 19a) umfassen, die zum Bestrahlen von Betriebsoberflächen des mindestens einen Behälterelements (14a, 14b, 14c) positioniert ist, um eine bakterielle Belastung auf den Betriebsoberflächen zu reduzieren, wobei die Betriebsoberflächen Oberflächen sind, die während des Betriebs der Ausrüstung (10) in Kontakt mit Eigelb und/oder Eiweiß kommen;
wobei die Förderer (16, 17) Folgendes aufweisen:
- mindestens einen ersten Förderer (16) mit einer Vielzahl von Sitzen, die jeweils zum Aufnehmen eines Eigelbs geeignet sind;
- mindestens einen zweiten Förderer (17) mit einer Vielzahl von Sitzen, die jeweils zum Aufnehmen von Eiweiß geeignet sind;
wobei jedes der Öffnungselemente (34) über einem des mindestens einen ersten Förderers (16) angeordnet ist, um ein Eigelb für jeden Sitz des ersten Förderers (16) zu entladen;
wobei jeder des mindestens einen ersten Förderers (16) über einem des mindestens einen zweiten Förderers (17) angeordnet ist, um Eiweiß in die Sitze des zweiten Förderers (17) zu entladen;
wobei die Betriebsoberflächen der Förderer (16, 17) die Sitze umfassen;
wobei die erste Desinfektionsvorrichtung (18, 19a) auch so angeordnet ist, dass sie die Sitze bestrahlt, bevor sie während des Betriebs der Ausrüstung (10) entsprechend mit einem Eigelb oder einem Eiweiß in Kontakt kommen.

2. Vorrichtung (10) nach Anspruch 1, wobei die Desinfektionsvorrichtungen (18, 19b, 20, 30) eine dritte Desinfektionsvorrichtung (20) umfassen, die zum Bestrahlen von Arbeitsteilen der Schälvorrichtung (12) angeordnet ist, wobei die Arbeitsteile Teile umfassen, die während des Betriebs der Ausrüstung (10) mit einem Ei in Kontakt kommen können.

3. Vorrichtung (10) nach einem der vorstehenden Ansprüche, wobei die erste Desinfektionsvorrichtung (18) neben der Schälvorrichtung (12) und stromaufwärts von letzterer in Bezug auf eine Vorschubrichtung (C) der Sitze angeordnet ist, um letztere kurz vor dem Befüllen zu desinfizieren.

4. Vorrichtung nach einem der vorstehenden Ansprüche, wobei die Entladevorrichtung (13) Folgendes umfasst
- ein Entleerungselement, das betreibbar ist, um mit dem zweiten Förderer (17) in einer ersten Entladeposition (F) der Entladekammer (B) zu interferieren; wobei das Entleerungselement dazu konfiguriert ist, nach seiner Betätigung ein Fallen des Inhalts der Sitze des ersten Förderers (16) zu bewirken, wenn sie die erste Entladeposition (F) während des Betriebs der Ausrüstung (10) passieren;
- ein erstes Behälterelement (14a) des mindestens einen Behälterelements (14a, 14b, 14c), das so positioniert ist, dass es bei Verwendung unter dem zweiten Förderer (17) in der ersten Entladeposition (F) angeordnet ist, um den Inhalt der Sitze des zweiten Förderers (17) aufzunehmen.

5. Vorrichtung (10) nach dem vorstehenden Anspruch, die eine erste Filtervorrichtung (27) umfasst, die so positioniert ist, dass sie bei Verwendung über dem ersten Behälterelement (14a) angeordnet ist, um Eierschalenfragmente zu filtern und zurückzuhalten und den Durchgang des Schälprodukts zu ermöglichen.

6. Vorrichtung (10) nach dem vorstehenden Anspruch, wobei eine zweite Desinfektionsvorrichtung (19a) der Desinfektionsvorrichtungen (18, 19a, 19b, 20, 30) der ersten Filtervorrichtung (27) und dem ersten Behälterelement (14a) zugewandt ist, um jegliche bakterielle Belastung auf diesen zu reduzieren.

7. Vorrichtung (10) nach einem der vorstehenden Ansprüche, wobei die Entladevorrichtung (13) Folgendes umfasst:
- einen Tank (28), der in der Entladekammer (B) so angeordnet ist, dass er sich bei Verwendung unter dem ersten Förderer (16) befindet, um das Schälprodukt von diesem aufzunehmen;
- eine Kühlvorrichtung, die thermisch mit dem Tank (28) verbunden ist, um ihn auf eine Temperatur von nicht mehr als 15° C und vorzugsweise zwischen 0° C und 4° C zu kühlen, beispielsweise mittels eines Kühlmantels mit Rückführung des Kühlmittels, der mit dem Tank (28) verbunden ist.

8. Vorrichtung (10) nach dem vorstehenden Anspruch, die dazu konfiguriert ist, das Schälprodukt von dem ersten Förderer (16) in eine zweite Entladeposition (G) der Entladekammer (B) zu entladen; wobei die Ausrüstung (10) eine zweite Filtervorrichtung (29) umfasst, die so angeordnet ist, um Schälprodukt von dem ersten Förderer (16), das in die zweite Entladeposition (G) entladen wird, aufzunehmen, um Eierschalenfragmente zu filtern und zurückzuhalten und den Durchgang des Schälprodukts zu ermöglichen.

9. Vorrichtung (10) nach dem vorstehenden Anspruch, die eine vierte Desinfektionsvorrichtung (30) der Desinfektionsvorrichtungen (18, 19a, 19b, 20, 30) umfasst, die der zweiten Filtervorrichtung (29) zugewandt ist, um jegliche bakterielle Belastung auf ihr zu reduzieren.

10. Vorrichtung (10) nach einem der Ansprüche 5 oder 6 und/oder nach einem der Ansprüche 8 oder 9, wobei die erste Filtervorrichtung (27) und/oder die zweite Filtervorrichtung (29) jeweils ein Netz oder ein perforiertes Förderband umfasst, das betreibbar ist, um während des Betriebs der Ausrüstung (10) gefilterte Eierschalenfragmente in eine Auswurfrichtung (H, J) zu ziehen.

11. Vorrichtung (10) nach einem der vorstehenden Ansprüche, die Belüftungsmittel umfasst, die in lufttechnischer Verbindung mit dem Inneren des Gehäuses (11) stehen, um ein Gas zu zwingen, in das Gehäuse zu strömen.

12. Vorrichtung nach Anspruch 11, wobei das Gas gefilterte Luft oder eine ozonhaltige gasförmige Mischung ist, wobei die gefilterte Luft aus einem durch einen HEPA-Filter gefilterten Luftstrom besteht.

13. Vorrichtung (10) nach einem der vorstehenden Ansprüche, umfassend eine Saugeinheit (32), die in lufttechnischer Verbindung mit dem Inneren des Gehäuses (11) steht und dazu konfiguriert ist, ein in dem Gehäuse enthaltenes Gas anzusaugen.

## Revendications

1. Appareil (10) pour le cassage d'oeufs incluant :
- un corps (11) délimitant un compartiment de cassage (A) et un compartiment de déchargement (B) ;
- un dispositif de cassage (12) logé dans ledit compartiment de cassage (A) et configuré pour ouvrir des coquilles d'oeufs ;
- un dispositif de déchargement (13) logé dans ledit compartiment de déchargement (B) et comprenant au moins un élément trémie (14a, 14b, 14c) approprié pour recevoir et décharger un produit de cassage constitué de blanc, de jaune, ou d'un mélange de blanc et de jaune ;
- un dispositif de transport (15) comprenant des convoyeurs (16, 17) configurés pour transporter séparément du blanc et du jaune dudit dispositif de cassage (12) audit dispositif de déchargement (13) ;
dans lequel ledit dispositif de cassage (12) comprend au moins un élément d'ouverture (34) configuré pour ouvrir une coquille d'oeuf et provoquer la chute du jaune et du blanc ;
**caractérisé en ce que** l'appareil inclut :
- des dispositifs de désinfection (18, 19a, 19b, 20, 30) logés dans ledit corps (11), conçus pour émettre un rayonnement ultraviolet et qui comprennent un premier dispositif de désinfection (18, 19a) positionné pour exposer des surfaces de travail dudit au moins un organe trémie (14a, 14b, 14c), afin de réduire une charge bactérienne sur lesdites surfaces de travail, lesdites surfaces de travail étant des surfaces qui entrent en contact avec du jaune et/ou du blanc durant le fonctionnement dudit appareil (10) ;
lesdits convoyeurs (16, 17) comprennent :
- au moins un premier convoyeur (16) ayant une pluralité de sièges conçus pour loger chacun un jaune ;
- au moins un deuxième convoyeur (17) ayant une pluralité de sièges appropriés pour loger chacun un blanc ;
dans lequel chacun dudit élément d'ouverture (34) est agencé au-dessus de l'un dudit ou desdits premiers convoyeurs (16) pour décharger un jaune pour chaque siège dudit premier convoyeur (16) ;
dans lequel chacun dudit ou desdits premiers convoyeurs (16) est agencé pardessus l'un dudit ou desdits deuxièmes convoyeurs (17) pour décharger du blanc dans les sièges dudit deuxième convoyeur (17) ;
les surfaces de travail desdits convoyeurs (16, 17) comprennent lesdits sièges ;
ledit premier dispositif de désinfection (18, 19a) étant également positionné de façon à exposer lesdits sièges avant qu'un jaune et un blanc respectivement ne s'engagent dedans, durant le fonctionnement dudit appareil (10).

2. Appareil (10) selon la revendication 1, dans lequel lesdits dispositifs de désinfection (18, 19b, 20, 30) comprennent un troisième dispositif de désinfection (20) positionné pour exposer des parties actives dudit dispositif de cassage (12), lesdites parties actives comprenant des parties susceptibles d'entrer en contact avec un oeuf durant le fonctionnement dudit appareil (10).

3. Appareil (10) selon n'importe laquelle des revendications précédentes, dans lequel ledit premier dispositif de désinfection (18) est agencé côte à côte avec ledit dispositif de cassage (12) et agencé en amont de ce dernier par rapport à un sens d'avancement (C) desdits sièges, pour désinfecter ces derniers juste avant qu'ils ne soient remplis.

4. Appareil selon l'une des revendications précédentes, dans lequel ledit dispositif de déchargement (13) comprend :
- un élément de vidage servant à interagir avec ledit deuxième convoyeur (17) dans une première position de déchargement (F) dudit compartiment de déchargement (B) ; ledit élément de vidage étant configuré pour provoquer, à la suite de son actionnement, une chute du contenu des sièges dudit premier convoyeur (16) lorsqu'ils passent par ladite première position de déchargement (F) durant le fonctionnement dudit appareil (10) ;
- un premier élément trémie (14a) dudit ou desdits éléments trémies (14a, 14b, 14c) qui est positionné de sorte qu'il se trouve, lors de l'utilisation, au-dessous dudit deuxième convoyeur (17) dans ladite première position de déchargement (F) pour recevoir le contenu des sièges dudit deuxième convoyeur (17).

5. Appareil (10) selon la revendication précédente, comprenant un premier dispositif de filtration (27) positionné de sorte qu'il se trouve, lors de l'utilisation, au-dessus dudit premier élément trémie (14a) pour filtrer et retenir des fragments de coquille d'oeuf et permettre le passage dudit produit de cassage.

6. Appareil (10) selon la revendication précédente, dans lequel un deuxième dispositif de désinfection (19a) desdits dispositifs de désinfection (18, 19a, 19b, 20, 30) fait face audit premier dispositif de filtration (27) et audit premier élément trémie (14a) pour réduite toute charge bactérienne présente sur eux.

7. Appareil (10) selon l'une des revendications précédentes, dans lequel ledit dispositif de déchargement (13) comprend :
- une cuve (28) agencée dans ledit compartiment de déchargement (B) de façon qu'elle se trouve, lors de l'utilisation, au-dessous dudit premier convoyeur (16) pour recevoir ledit produit de cassage provenant de ce dernier ;
- un dispositif de réfrigération relié thermiquement à ladite cuve (28) pour la refroidir à une température ne dépassant pas 15 °C et de préférence comprise entre 0 °C et 4 °C, par exemple au moyen d'une chemise de refroidissement à recirculation de fluide frigorigène associée à la cuve (28).

8. Appareil (10) selon la revendication précédente, configuré pour décharger ledit produit de cassage provenant dudit premier convoyeur (16) dans une deuxième position de déchargement (G) dudit compartiment de déchargement (B) ; ledit appareil (10) comprenant un deuxième dispositif de filtration (29) agencé de façon à recevoir un produit de cassage provenant dudit premier convoyeur (16) déchargé dans ladite deuxième position de déchargement (G), pour filtrer et retenir des fragments de coquille d'oeuf et permettre le passage dudit produit de cassage.

9. Appareil selon la revendication précédente, comprenant un quatrième dispositif de désinfection (30) desdits dispositifs de désinfection (18, 19a, 19b, 20, 30) qui fait face audit deuxième dispositif de filtration (29) pour réduire toute charge bactérienne présente sur lui.

10. Appareil (10) selon l'une des revendications 5 et 6 et/ou selon l'une des revendications 8 et 9, dans lequel ledit premier dispositif de filtration (27) et/ou ledit deuxième dispositif de filtration (29), respectivement, comprennent une bande transporteuse à mailles ou perforée servant à entraîner, dans une direction d'éjection (H, J), des fragments de coquille d'oeuf filtrés durant le fonctionnement dudit appareil (10).

11. Appareil (10) selon l'une des revendications précédentes, comprenant des moyens de ventilation qui sont en communication aéraulique avec l'intérieur dudit corps (11) pour forcer un gaz à rentrer dans ce dernier.

12. Appareil selon la revendication 11, dans lequel ledit gaz est de l'air filtré ou un mélange gazeux contenant de l'ozone ; ledit air filtré étant constitué d'un écoulement d'air filtré par un filtre HEP A.

13. Appareil (10) selon l'une des revendications précédentes, comprenant une unité d'aspiration (32) qui est en connexion aéraulique avec l'intérieur dudit corps (11) et est configurée pour aspirer un gaz contenu dans ce dernier.
